# FASCICULE DE BREVET EUROPEEN

(11) **EP 1 646 872 B1**
(45) Date de publication et mention de la délivrance du brevet: **12.11.2008**
(21) Numéro de dépôt: 04767619.2
(22) Date de dépôt: 08.07.2004
(51) Int. Cl.: G01N 33/569, C07H 21/00

(54) **DOSAGE DES ACIDES TECHOIQUES DES BACTERIES GRAM+**
DOSIERUNG VON TEICHONSÄUREN VON GRAM+-BAKTERIEN
DOSING OF TEICHOIC ACIDS OF GRAM+ BACTERIA

(30) Priorité: 08.07.2003 FR 0308305
(43) Date de publication de la demande: 19.04.2006
(73) Titulaire: Sanofi Pasteur, 69367 Lyon Cedex 07 (FR)
(72) Inventeur: TALAGA, Philippe, F-69280 Saint-Consorce (FR); SEPULCRI, Patricia, F-69290 Saint Genis Les Ollières (FR); VIALLE-BLANC, Sandrine, Rés. Les Jardins d'Eva, F-69290 Craponne (FR)
(74) Mandataire: Hurpin, Christian Marcel
(86) Numéro de dépôt international: PCT/FR2004/001787
(87) Numéro de publication internationale: WO 2005/005988

(56) Documents cités:
- TALAGA P ET AL: "Development of a high-performance anion-exchange chromatography with pulsed-amperometric detection based quantification assay for pneumococcal polysaccharides and conjugates" VACCINE, BUTTERWORTH SCIENTIFIC. GUILDFORD, GB, vol. 20, no. 19-20, 7 juin 2002 (2002-06-07), pages 2474-2484, XP004359677 ISSN: 0264-410X cité dans la demande
- JENNINGS H J ET AL: "FACILE CLEAVAGE OF SOME 2-ACETAMIDO-2-DEOXY-BETA-D-GLUCO- AND GALACTOPYRANOSIDE USING AQUEOUS HF" CANADIAN JOURNAL OF CHEMISTRY, NATIONAL RESEARCH COUNCIL. OTTAWA, CA, vol. 58, 1980, pages 2610-2612, XP001189608 ISSN: 0008-4042 cité dans la demande
- KARLSSON C ET AL: "THE PNEUMOCOCCAL COMMON ANTIGEN C-POLYSACCHARIDE OCCURS IN DIFFERENT FORMS MONO-SUBSTITUTED OR DI-SUBSTITUTED WITH PHOSPHOCHOLINE" EUROPEAN JOURNAL OF BIOCHEMISTRY, BERLIN, DE, vol. 265, 1999, pages 1091-1097, XP001193753 ISSN: 0014-2956 cité dans la demande

## Description

L'invention a pour objet un procédé de dosage des acides téchoïques de type polysaccharidique dans une préparation issue de culture de streptococcus pneumoniae.

Les acides téchoïques sont des constituants du complexe constitué de la membrane et de la paroi des bactéries Gram+. Ils sont en contact étroit avec le peptidoglycane de la paroi. Des liaisons covalentes renforcent ce lien. Les acides téchoïques qui comportent dans leur structure chimique une extrémité lipidique ou glycolipidique peuvent s'ancrer dans la membrane par liaison hydrophobe.

On distingue principalement deux types d'acides téchoïques :
- le premier type regroupe les acides téchoïques qui sont des polymères linéaires de ribitol phosphate ou de glycérol phosphate unis par des liaisons phosphodiesters. Les fonctions hydroxyles libres des ribitols ou des glycérols peuvent être substituées par des résidus tels que des résidus d'acides aminés, de N acétyl glucosamine, de glucose, ou de N acétyl galactosamine. Ces acides téchoïques sont répertoriés comme des « polymères d'alcool ».
- le deuxième type regroupe les acides téchoïques qui sont des polysaccharides formés d'un enchaînement d'unités répétitives. L'unité répétitive est généralement constituée d'un enchaînement d'au moins deux monosaccharides suivis d'un ribitol phosphate ou d'un glycérol phosphate. Le groupement phosphate intervient dans l'enchaînement des unités répétitives au moyen de liaisons phosphodiesters. Les fonctions hydroxyles libres des unités répétitives peuvent être également substituées par des résidus divers.

De plus, parmi les acides téchoïques, on distingue les acides lipotéchoïques qui possèdent une chaîne lipidique ou glycolipidique additionnelle. Cette dernière est reliée au ribitol phosphate ou au glycerol phosphate terminal au moyen d'une liaison phosphodiester. La partie lipidique est habituellement une chaîne de monoacyl glycérol ou de diacyl glycérol.

Selon les espèces bactériennes, les acides téchoïques sont de type « polymère d'alccol » comme dans le cas de *Staphylococcus aureus* ou de type « polysaccharidique » comme dans le cas de *Streptococcus pneumoniae.* Dans cette espèce, on connaît deux acides téchoïques ubiquitaires : le polysaccharide C et l'acide lipotéchoïque (encore dénommé antigène de Forssmann).

Le polysaccharide C est constitué d'un enchaînement d'unités répétitives, chacune formée de quatre résidus monosaccharidiques, (un résidu de glucopyrannose (Glcp), un résidu de 2 acétamido-4-amino-2,4,6-trideoxy-D galactose (AAT), et deux résidus de N-acétyl galactopyrannose (Gal*p*NAc)), suivis d'un résidu de ribitol-5-phosphate (ribitol-5-*P*). Au moins un des deux résidus de Gal*p*NAc est substitué par de la phosphocholine (*P*-Cho) La formule développée de cette unité répétitive est la suivante :

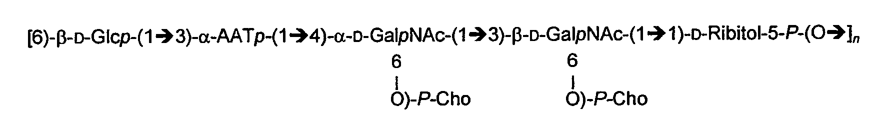

C Karlsson et coll., Eur. J. Biochem. (1999) 265 : 1091 ont montré que l'unité répétitive pouvait exister sous deux formes : (i) une forme bi-substituée avec deux résidus phosphocholine, comme représentée ci dessus ; et (ii) une forme monosubstituée avec un seul résidu de phosphocholine. Par conséquent, la chaîne du polysaccharide C contient en proportion variable des unités répétitives monosubstituées et bi-substituées. Ces proportions varient notamment en fonction des conditions de culture et du sérotype du pneumocoque.

L'acide lipotéchoïque de *Streptococcus pneumoniae,* quant à lui, possède la structure primaire du polysaccaride C associée à un glycolipide, de formule β-D-Glc*p*(1-4)-β-D-AAT (1-3)- α-D-Glcp(1-3)acyl₂Gro, dans laquelle Glc*p* désigne un résidu de glucopyrannose, AAT désigne un résidu de 2 acétamido-4-amino-2,4,6-trideoxy-D-galactose, acyl désigne un résidu d'acide gras et Gro un résidu de glycérol. Ce glycolipide est lié par liaison phosphodiester au ribitol terminal de la chaîne.

Certaines bactéries Gram+, telles *Streptococcus pneumoniae,* possèdent une capsule et/ou sont responsables de graves infections. A l'encontre de *Streptococcus pneumoniae*, il existe des vaccins constitués par des polysaccharides de capsule sous forme purifiée. Les procédés utilisés pour purifier les polysaccharides capsulaires éliminent autant que possible les polysaccharides de la paroi (acides téchoïques). En effet, bien que très immunogènes, les acides téchoïques sont faiblement protecteurs et leur présence dans un vaccin augmente inutilement la charge antigénique. Par ailleurs, les acides téchoïques peuvent déclencher des réactions inflammatoires indésirables (Infection & Immunity (2003) 71 : 5541)

De nos jours, les autorités de santé exigent que le contenu d'un vaccin de haute qualité soit caractérisé avec précision. Il faut pouvoir doser non seulement l'antigène vaccinal mais aussi tous les contaminants éventuels, au rang desquels se trouvent les acides téchoïques, notamment pour ce qui concerne les vaccins à base de polysaccharides capsulaires. Le dosage des contaminants est d'autant plus difficile et délicat qu'il porte sur de très petites quantités. Les méthodes de dosage doivent donc être sensibles, performantes et spécifiques.

Cette dernière exigence de spécificité est particulièrement difficile à respecter. En effet, il existe de fortes analogies de structure entre les acides téchoïques de type polysaccharidique et les polysaccharides de capsule. Par exemple, l'unité répétitive du polysaccharide C de *Streptococcus pneumoniae* contient un groupement phosphate tout comme celle des polysaccharides capsulaires des sérotypes 6B, 10A, 11A, 15B, 17F, 18C, 19A, 19F, 20, 23F. Le groupement phosphate de l'unité répétitive du polysaccharide C intervient directement dans l'enchaînement des unités répétitives par l'intermédiaire d'une liaison phosphodiester, tout comme ceux des polysaccharides capsulaires de sérotype 6B, 10A, 17F, 19A, 19F et 20. Les polysaccharides capsulaires des sérotypes 6B et 10A sont ceux qui présentent la plus forte analogie avec les acides téchoïques du pneumocoque dans la mesure où ils possèdent un ribitol phosphate impliqué dans l'enchaînement des unités répétitives.

Jones C. et coll. ont décrit dans Biologicals (1991) 19 : 41 une méthode de dosage du polysaccharide C par résonance magnétique nucléaire (RMN). Cette méthode évalue la résonance du radical N-méthyl des groupements phosphocholine du polysaccharide C.

Cette technique n'est pas précise car, comme indiqué plus haut, la proportion des groupements phosphocholine n'est pas constante.

Talaga P. et coll. proposent dans Vaccine (2001) 19 : 2987 une méthode de dosage basée sur la quantification du ribitol libéré lors de l'hydrolyse du polysaccharide C à la suite de deux traitements successifs : le premier avec l'acide fluorhydrique (HF) à 48 % pendant 2 heures à 65°C, le deuxième avec l'acide trifluoroacétique 2N pendant 2 heures à 120°C. Ce double traitement entraîne la rupture des liaisons osidiques. L'hydrolysat contient essentiellement des monosaccharides. Le ribitol est ensuite séparé des autres constituants par chromatographie haute performance échangeuse d'anions (HPAEC) sur une colonne de chromatographie analytique CarboPac^{™} MA1 en utilisant pour l'élution, une solution de soude isocratique 480 mM. Le ribitol est ensuite quantifié par un système de détection ampérométrique en champs pulsés (PAD). Même si cette technique de dosage du ribitol permet une évaluation juste et sensible de la quantité de polysaccharide C, elle est incompatible avec la présence dans le milieu, d'un polysaccharide capsulaire qui contient du ribitol.

Or, certains polysaccharides capsulaires d'intérêt vaccinal contiennent du ribitol. Comme on vient de le voir, il s'agit par exemple des polysaccharides de capsule des sérotypes 6B et 10A de *Streptococcus pneumoniae.* La technique de Talaga et coll. ne convient donc pas pour effectuer le dosage des acides téchoïques dans une préparation qui contient ces sérotypes, *e*.*g*. dans les vaccins pneumocoque du commerce.

En bref, les méthodes de dosage des acides téchoïques de type polysaccharidique connues à ce jour présentent des inconvénients. Il n'existe pas encore de méthode qui soit précise, sensible, fiable et applicable à toute préparation issue de culture de bactéries Gram+,en particulier les préparations issues de la culture de *Streptococcus pneumoniae.*

Les pneumocoques (*Streptococcus pneumoniae*) sont des bactéries Gram+ capsulées, responsables de pathologies infectieuses notamment de méningites, de bronchites, de rhinites et otites à complications chez l'adulte comme chez l'enfant. Les pneumocoques sont divisés en sérotypes selon la structure des polysaccharides qui forment la capsule. Le sérotypage des pneumocoques est réalisé à l'aide d'une batterie d'immun-sérums, chaque immun-sérum étant spécifique d'un seul type de polysaccharide capsulaire (immun-sérums monospécifiques). Plus de 90 sérotypes différents ont été recensés jusqu'à présent.

Les vaccins contre le pneumocoque actuellement commercialisés contiennent tous des polysaccharides de capsule. La purification des polysaccharides étant réalisée à partir d'un lysat de bactéries, ces vaccins contiennent des quantités résiduelles d'acides téchoïques qu'il convient de doser.

Les polysaccharides capsulaires de *Streptococcus pneumoniae* sont notamment choisis parmi ceux qui caractérisent les 23 sérotypes (valences) habituellement rencontrés chez l'homme ; Soient les sérotypes 1, 2, 3, 4, 5, 6B, 7F, 8, 9N, 9V, 10A, 11A, 12F, 14, 15B, 17F, 18C, 19A, 19F, 20, 22F, 23F et 33F.

La présente invention pallie ce manque en proposant une nouvelle méthode de dosage des acides téchoïques de type polysaccharidique dans une préparation issue de la culture de *Steptococcus pneumoniae*. Cette méthode recherche les oligosaccharides spécifiques des acides téchoïques libérés par rupture des liaisons phosphodiester, au cours du traitement par l'acide fluorhydrique (HF), selon la méthode de Jennings & Lugowski, Can. J. Chem. (1980) 58 : 2610. Lorsque l'unité répétitive renferme une molécule de β-D GalpNac ou une molécule de β-D GlcpNac liée à une molécule de ribitol phosphate ou de glycerol phosphate, il peut également se produire une rupture de cette liaison avec libération du ribitol ou du glycerol. Autrement dit, selon la structure de l'acide téchoïque, l'oligosaccharide spécifique sera substantiellement identique à l'unité répétitive ou bien s'en distinguera par perte du ribitol ou du glycérol.

Ainsi, dans le cas du polysaccharide C, l'oligosaccharide spécifique a pour formule :

β-D-Glcp-(1**→**3)-α**-**AAT*p***-**(1**→**4)-α-D-Gal*p*NAc-(1**→**3)-β-D-Gal*p*NAc

Après hydrolyse, restent alors des oligosaccharides spécifiques qui sont par la suite séparés les uns des autres et dosés.

Par ailleurs, on indique par souci d'exhaustivité, que cette définition s'applique *mutatis mutandis* aux oligosaccharides spécifiques des polysaccharides capsulaires possédant des unités répétitives incorporant des groupements phosphate impliqués dans la chaîne, tels les polysaccharides des sérotypes 6B, 10A, 17F, 19A, 19F et 20 du pneumocoque.

Ainsi la présente invention a pour objet une méthode de dosage des acides téchoïques de type polysaccharidique dans une préparation issue de culture de *Streptococcus pneumoniae,* selon laquelle:
(1)on traite la préparation par l'acide fluorhydrique (HF) à une température inférieure ou égale à 40°C, afin de libérer les oligosaccharides spécifiques des acides téchoïques présents dans la préparation, dont la structure de ces oligosaccharides est dépourvue de toute liaison chimique de type phosphate ester mais est identique à l'enchaînement des sucres de l'unité répétitive des acides téchoïques ou s'en distingue par la perte du ribitol ou du glycérol lorsque la structure de l'unité répétitive renferme une molécule de β-D GalpNAc ou une molécule de β-D GlcpNAc liée à une molécule de ribitol phosphate ou de glycerol phosphate; et
(2) on dose les oligosaccharides spécifiques obtenus en *(1)*.

Elle concerne notamment une méthode de dosage selon laquelle les acides téchoïques à doser sont le polysaccharide C et l'acide lipotéchoïque et selon laquelle les oligosaccharides spécifiques du polysaccharide C et de l'acide lipotéchoïque ont une structure chimique commune sous la forme :

β-D-Glcp-(1**→**3)-α**-**AAT*p***-**(1**→**4)-α-D-Gal*p*NAc-(1**→**3)-β-D-Gal*p*NAc

Par « préparation issue de culture de*Streptococcus pneumoniae* » on entent : (i) une préparation de bactéries entières ; (ii) un lysat de bactéries que l'on obtient notamment en traitant les bactéries avec des détergents chimiques (désoxycholate, tween, éther, et...) ou en utilisant des procédés mécaniques tels que le choc osmotique ou la sonication ; (iii) un produit de fractionnement des bactéries que l'on obtient par exemple en utilisant le procédé de précipitation alcoolique fractionnée ; (iv) un surnageant de culture de bactéries ; et (v) une préparation comprenant un ou plusieurs composants (antigènes) de bactéries, sous forme purifiée.

De façon préférée, la préparation de *Streptococcus pneumoniae* est un surnageant de culture de *Streptococcus pneumoniae* ou une préparation comprenant un ou plusieurs antigènes purifiés ; cette dernière étant tout particulièrement préférée.

Le traitement par l'acide fluorhydrique (HF) est avantageusement mis en oeuvre à une température allant de -70°C à 40°C, de préférence allant de 0°C à 20°C, de façon encore plus préférée ente 4°C et 10°C. D'une manière générale, plus la température augmente, plus la sélectivité d'action de l'acide fluorhydrique sur les liaisons phosphodiesters diminue. C'est pourquoi, il est important de ne pas dépasser 40°C.

De manière typique, on utilise l'acide fluorhydrique à une concentration finale comprise entre 10 et 73 % (poids / poids), bornes incluses ; de préférence entre 40 et 60 % ; de manière tout à fait préférée entre 45 et 50 % ; *e*.*g*. à une concentration de 48 %.

La durée du traitement à l'acide fluorhydrique n'est pas critique. Elle est inversement proportionnelle à la température. L'homme du métier est à même d'adapter cette durée en fonction de la température choisie et de la concentration à laquelle est utilisé l'acide fluorhydrique. Toutefois, on indique que plus la température est élevée, plus la durée du traitement doit être courte pour assurer autant que possible l'intégrité des liaisons osidiques. D'une manière générale, il est préférable que le traitement n'excède pas 96 heures. Lorsque la température est comprise entre 20 et 40°C, on préconise une durée de traitement n'excédant pas 1 heure ; entre 0 et 10°C, n'excédant pas 2 heures. A l'inverse lorsque la température est inférieure à 0°C, la durée du traitement peut dépasser 24 heures.

De bons résultats sont obtenus lorsque l'on utilise l'HF à une concentration de 48 %, à 4°C pendant 48 heures. Dans ces conditions, les taux de recouvrement en oligosaccharide spécifique peuvent atteindre 90 % de la valeur théorique.

Pour doser le(s) oligosaccharide(s) spécifique(s) des acides téchoïques obtenus après traitement avec l'HF, il convient avantageusement de le(s) séparer des autres constituants et de le(s) caractériser. Séparation, caractérisation et quantification peuvent être mises en oeuvre selon différentes techniques, *e*.*g*. biochimiques, accessibles à l'homme du métier. On indique toutefois que les méthodes connues pour permettre la séparation et/ou le dosage des monosaccharides sont tout particulièrement appropriées.

Selon un mode particulier, on a recours à une technique de chromatographie par échange d'anions hautement résolutive (HPAEC), éventuellement couplée à une détection ampérométrique en champs pulsés (PAD).

A cette fin, un support de chromatographie approprié doit permettre une bonne résolution et être compatible avec un milieu de pH très élevé (≥ 12). En pratique, dans ces conditions de pH, le support doit rester intact, exempt de dégradation. Il peut être constitué par une résine *e*.*g*., de polystyrène-divinyl-benzène sulfonaté, ayant par exemple un degré de réticulation variant entre 1 et 5 %. La résine est avantageusement sous forme de microbilles dont le diamètre varie de préférence entre 450 et 550 nm. Si le milieu issu du traitement à l'HF contient de nombreux produits, il peut être utile d'optimiser la résolution. A cette fm, la résine *e*.*g*., de polystyrène-divinyl-benzène sulfonaté peut être conditionnée sous forme de colonne et être complétée en sa partie supérieure par une couche d'un matériau porteur de charges positives, *e*.*g*., d'un sel d'amine primaire, d'un sel d'amine secondaire, d'un sel d'amine tertiaire, d'un sel d'ammonium quaternaire, ou d'un groupement ammonium. Ce matériau peut se présenter sous forme de billes ayant avantageusement un diamètre de 5 à 15 µm, *e*.*g*. 10 µm environ. Ce matériau peut être poreux ou non. A titre d'exemple on indique qu'un matériau approprié peut être constitué par du latex ayant avantageusement un degré de réticulation de 3 à 7 %, *e*.*g*., 5 % environ.

Le matériel de chromatographie (*e*.*g*. colonne analytique) commercialisé par les sociétés Dionex et Metrohm sous les marques de commerce respectives CarboPac^{™} et Metrosep Carb répond aux critères requis. Ce matériel est couramment désigné comme étant « de type CarboPac^{™} ». La colonne CarboPac^{™} PA1 est tout particulièrement préférée.

Une fois la préparation issue du traitement par l'HF déposée sur la colonne de chromatographie, on élue avec une solution d'élution possédant un pH ≥ 12, notamment pour que les fonctions hydroxyles des sucres s'ionisent et soient sous forme d'oxyanions séparables par chromatographie d'échange d'anions. Si la technique PAD est utilisée par la suite pour la détection, la solution d'élution doit être aussi compatible avec cette dernière. A cet égard, la solution d'élution est avantageusement dépourvue de carbonate.

De manière avantageuse, la solution d'élution est une solution de soude dont la molarité est comprise entre 10 et 300 mM, de manière préférée entre 20 et 150 mM, de façon encore plus préférée entre 40 et 100 mM.

Le débit de 1a solution d'élution est fonction du type de colonne utilisée, mais est généralement compris entre 0.1 et 4 ml/min.

Les oligosaccharides spécifiques sont décelés sur le chromatogramme sous la forme de pics de chromatographie ayant des temps de rétention caractéristiques dans des conditions opératoires déterminées (même colonne de chromatographie, même solution et vitesse d'élution, etc).

Enfin on recherche et on quantifie l'oligosaccharide spécifique de l'acide téchoïque que l'on cherche à doser à l'aide d'un système de détection, par exemple par ampérométrie en champs pulsés (PAD). Cette méthode de détection est basée sur l'oxydation des sucres sur une électrode de travail entraînant la formation d'un courant électrique qui est mesuré. Des potentiels de régénération et de nettoyage sont souvent appliqués à l'électrode de travail.

Pour connaître la quantité absolue de l'acide téchoïque que l'on cherche à doser, on se reporte avantageusement à une courbe d'étalonnage établie à partir d'une préparation purifiée de l'acide téchoïque en question, dans les mêmes conditions de traitement et d'analyse.

Grâce à la méthode de dosage selon l'invention, il est en particulier possible de quantifier les acides téchoïques du pneumocoque dans n'importe quelle préparation contenant un ou plusieurs polysaccharide(s) capsulaire(s), quels que soient le nombre et le sérotype. En particulier, la préparation peut contenir un ou plusieurs polysaccharides capsulaires purifiés sous une forme native ou modifiée par dépolymérisation partielle, activation ou conjugaison à un peptide ou une protéine porteuse comme l'anatoxine tétanique ou l'anatoxine diphtérique dont la structure présente des analogies avec celles des acides téchoïques. Il s'agit des polysaccharides des sérotypes 6B, 10A, 17F, 19A, 19F et 20. En effet, la présence de ces polysaccharides dont l'hydrolyse dans les conditions de l'invention entraîne la production d'oligosaccharides spécifiques, est sans incidence sur le dosage des acides téchoïques.

La méthode selon l'invention s'applique donc au dosage des acides téchoïques dans une préparation comprenant un ou plusieurs polysaccharides capsulaires des sérotypes 6B, 10A, 17F, 19A, 19F ou 20.

La préparation peut aussi contenir un ou plusieurs polysaccharides des sérotypes 11A, 15B, 18C et 23F. En bref, la préparation peut contenir les polysaccharides des 23 sérotypes les plus répandus ou n'importe quelle combinaison possible à partir de ces 23 sérotypes. A titre d'exemple, une telle préparation peut contenir les polysaccharides des sérotypes 4, 6B, 9V, 14, 18C, 19F et 23F. Elle peut aussi contenir un ou plusieurs polysaccharides additionnels provenant des sérotypes 1, 3, 5 et 7F.

A titre indicatif, on soumet les remarques suivantes :
- Les oligosaccharides spécifiques du polysaccharide C et de l'acide lipotéchoïque libérés lors du traitement par HF à froid selon l'invention, sont identiques. En effet, les deux acides téchoïques possèdent la même unité répétitive. On ne peut donc pas doser le polysaccharide C et l'acide lipotéchoïque séparément.
- Les polysaccharides capsulaires des sérotypes 6B, 10A, 17F, 19A, 19F et 20, dont l'enchaînement des unités répétitives fait intervenir des liaisons phosphodiesters, sont dégradés essentiellement sous la forme d'oligosaccharides spécifiques.
- Les polysaccharides capsulaires des sérotypes 11A, 15B, 18C et 23F, dont les liaisons phosphodiesters ne sont pas directement impliquées dans l'enchaînement des unités répétitives, ne peuvent pas être hydrolysés sous forme d'oligosaccharides spécifiques. Soumis au traitement à l'HF, ils peuvent néanmoins se dépolymériser de manière partielle et aléatoire en des produits hétérogènes, incluant en particulier des molécules tels que le glycérol ou la choline, et des monosaccharides.
- Enfin les autres polysaccharides capsulaires qui ne contiennent pas de groupements phosphate ne sont pas dépolymérisés ou sont dépolymérisés de façon partielle et aléatoire pour donner après traitement à l'HF, des produits hétérogènes, en majorité de haut poids moléculaire, non-identifiables et non-quantifiables, ainsi qu'une minorité de monomères et des monosaccharides.

En définitive, le milieu issu du traitement à l'HF peut présenter une complexité plus ou moins importante en fonction du nombre et du type de polysaccharide(s) capsulaire(s) présent(s) au départ. Il convient donc de séparer l'oligosaccharide spécifique des acides téchoïques, des autres produits d'hydrolyse (monomères, monosaccharides, polysaccharides non hydrolysés ou partiellement hydrolysés ...).

De façon surprenante, l'oligosaccharide spécifique des acides téchoïques de *Streptococcus pneumoniae* se comporte comme un monosaccharide lorsqu'il est analysé et séparé par la technologie HPAEC-PAD et se distingue des oligosaccharides des polysaccharides capsulaires. Pour le séparer et le quantifier par HPAEC-PAD de façon particulièrement résolutive, on recommande l'utilisation d'une colonne de chromatographie CarboPac PA1^{™} et une solution de soude isocratique 75 mM. Dans ces conditions, le pic de chromatographie correspondant a un temps de rétention de 4,30 min ± 10 % lorsque le débit de la solution d'élution est de 1 ml/min. La surface du pic de chromatographie reflète la quantité relative de polysaccharide C et d'acide lipotéchoïque présents dans la préparation.

En utilisant la méthode de dosage des acides téchoïques selon invention, on a découvert que *S. pneumoniae* de sérotype 5 ne possédait pas de polysaccharide C. Par contre, on trouve un acide téchoïque de substitution que l'on a appelé polysaccharide C5. La formule de son unité répétitive diffère légèrement de celle du polysaccharide C. Elle est la suivante :

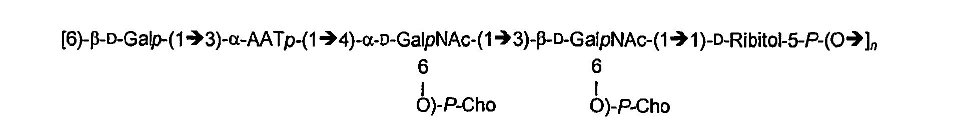

C'est pourquoi selon un mode très particulier, l'invention s'applique également au dosage du polysaccharide C5, notamment dans une préparation contenant le polysaccharide capsulaire du sérotype 5 de *S. pneumoniae.* Le mode opératoire particulièrement résolutif décrit ci-avant pour le dosage du polysaccharide C convient également pour le dosage du polysaccharide C5. Son pic de chromatographie a dans ces conditions un temps de rétention de 3,30 min + 10 %.

Pour déterminer les quantités absolues résiduelles en acides téchoïques, on se réfère à des courbes-étalon réalisées à partir de préparations « mère » exprimant des quantités connues en fonction des surfaces des pics de chromatographie.

Par exemple, les quantités absolues résiduelles en polysaccharide C et en acide lipotéchoïque, peuvent être déterminées en utilisant une préparation de polysaccharide C purifiée, telle que celle fournie par Staten Serum Institute, Danemark (cf exemple1).

Pour doser les quantités absolues résiduelles en polysaccharide C5, on utilise de préférence, comme préparation mère, une préparation purifiée de polysaccharide C5 préparée à partir d'un lysat de pneumocoques de sérotype 5. On extrait dans un premier temps les polysaccharides par une ou plusieurs précipitations alcooliques fractionnées. On complète la purification en éliminant les principaux contaminants, *i*.*e*. les protéines et les acides nucléiques ; puis on soumet ensuite l'extrait polysaccharidique débarrassé de ces principaux contaminants à une chromatographie de partage ou à une chromatographie échangeuse d'ions de façon à séparer le polysaccharide C5 des autres polysaccharides. On peut notamment utiliser le procédé chromatographique tel que décrit dans l'exemple 1. Les procédés d'extraction des polysaccharides par précipitation alcoolique fractionnée, de même que les procédés d'élimination des contaminants protéiques et d'acides nucléiques sont des procédés bien connus de l'homme de métier. On pourra notamment se référer aux procédés décrits dans US 4,686,102.

La préparation de polysaccharide C5 ainsi purifiée peut servir d'étalon pour déterminer les quantités résiduelles absolues de polysaccharide C5 par la méthode selon l'invention ; mais plus généralement, elle peut être utilisée dans n'importe quelle méthode de dosage du polysaccharide C5 qui se réfère à une courbe d'étalonnage.

L'invention a donc finalement pour objet l'utilisation d'un polysaccharide C5 purifié possédant une unité répétitive de formule : 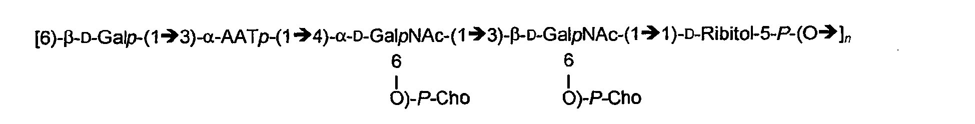 pour établir une courbe d'étalonnage destinée au dosage du polysaccharide C5, dans une préparation contenant un polysaccharide capsulaire de *Streptococcus pneumoniae* de sérotype 5.

La présente invention sera mieux comprise à la lumière des exemples suivants qui servent à illustrer l'invention sans pour autant en limiter le contenu.

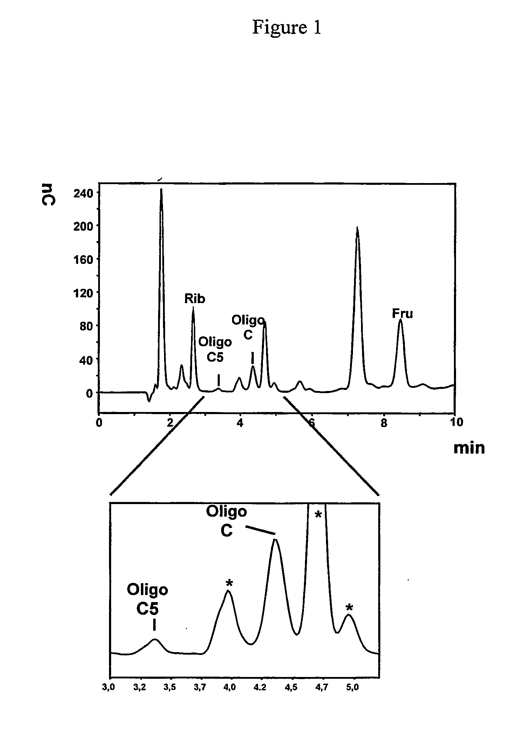

La figure 1 représente le chromatogramme HPAEC-PAD, après traitement à l'HF, du vaccin Pneumo 23^{™} contenant les polysaccharides capsulaires des sérotypes 1, 2, 3, 4, 5, 6B, 7F, 8, 9N, 9V, 10A, 11A, 12F, 14,15B, 17F, 18C, 19A, 19F, 20, 22F, 23F et 33F. Les pics surmontés d'une * correspondent aux monosaccharides provenant de l'hydrolyse des polysaccharides capsulaires. Le pic Rib correspond au ribitol. Le pic Fru correspond au fructose (étalon-interne). Les pics oligo C et oligo C5 et correspondent respectivement aux oligosaccharides spécifiques C et C5.

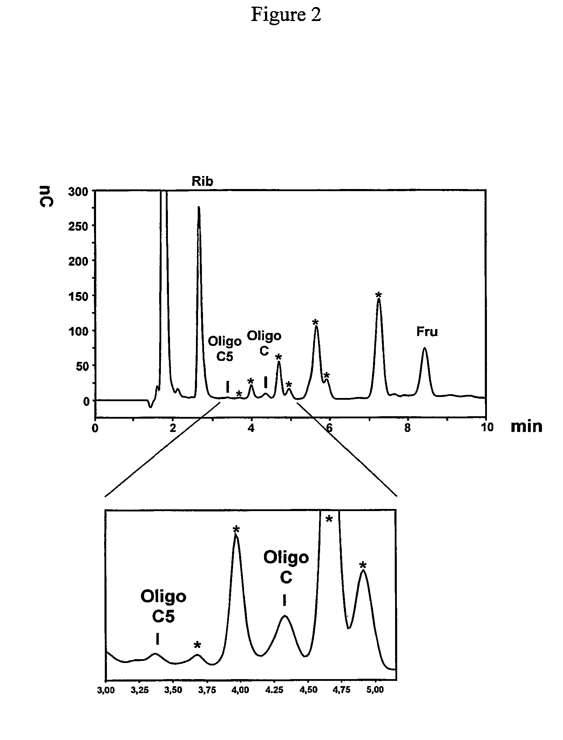

La figure 2 représente le chromatogramme HPAEC-PAD, après traitement à l'HF, d'une formulation vaccinale F3 telle que décrite dans WO 98/51339 contenant les conjugués Tt et Dt des polysaccharides capsulaires des sérotypes 1, 3, 4, 5, 6B, 7F, 9V, 14, 18C, 19F et 23F du pneumocoque.

### Exemple 1 : Dosage des quantités résiduelles en polysaccharide C et C5 dans le vaccin Pneumo 23^{™} contenant les polysaccharides capsulaires des sérotypes 1, 2, 3, 4, 5, 6B, 7F, 8. 9N, 9V, 10A, 11A, 12F, 14, 15B, 17F, 18C, 19A, 19F, 20, 22F, 23F et 33F du pneumocoque.

### 1.1. Solutions mère pour gamme étalon.

### 1.1.1. Polysaccharide C5 :

Ce dernier est purifié comme décrit dans le paragraphe suivant, à partir d'une préparation de polysaccharides du pneumocoque de sérotype 5 obtenue par précipitation alcoolique fractionnée d'un lysat de pneumocoques de sérotype 5, suivie d'une extraction phénolique pour éliminer les protéines, d'une précipitation fractionnée en présence de chlorure de calcium pour éliminer les acides nucléiques, et finalement d'une seconde précipitation alcoolique fractionnée. Le précipité obtenu, est lavé à l'alcool absolu puis séché sous vide. Le dessicat est remis en solution à une concentration de 10 mg/ml dans un tampon NaCl 200 mM .

On dépose 4 ml de cette solution sur une colonne de chromatographie (90 cm x 1,6 cm de diamètre) contenant du gel de sépharose-CL 4B. On soumet la colonne à un flux d'une solution de NaCl 200 mM à un débit de 0,6 ml/min de manière à séparer le polysaccharide C5 du polysaccharide capsulaire. L'éluat est recueilli en fractions. La mesure de l'absorbance UV à 206 nm de chaque fraction est mesurée. Cela permet d'identifier deux séries de fractions bien distinctes : la première contenant le polysaccharide capsulaire de type 5 ; la seconde, éluée contenant le polysaccharide C5. Cette seconde série de fractions est dialysée contre de l'eau distillée, concentrée à l'aide d'un évaporateur rotatif, puis conservée sous forme de lyophilisat. Le taux de pureté du lyophilisat est d'environ 70 %.

Par la suite on détermine la quantité exacte de polysaccharide C5 dans la préparation ainsi obtenue, en dosant le ribitol libéré après hydrolyse.

Le lyophilisat est remis en solution en eau purifiée ultrafiltrée à raison de 10 µg/ml (poids sec).

En parallèle, on réalise une gamme-étalon de ribitol de 0 à 4 µg/ml dans de l'eau purifiée ultrafiltrée. Pour contrôler la reproductibilité de la chromatographie, on peut rajouter à tous les échantillons une quantité fixe de mannose qui joue le rôle d'étalon interne.

400 µl de la solution de polysaccharide C5 préparée ci-dessus sont séchés sous azote. Le dessicat est traité par 200 µl d'acide fluorhydrique à 48 % pendant 2 heures à 65°C. On sèche et on rajoute 400 µl d'acide trifluoroacétique 2 N pendant 2 heures à 135°C. On sèche sous courant d'azote.

Pour analyse par chromatographie HPAEC-PAD, on dissout les dessicats obtenus à l'étape précédente dans 400 µl d'eau purifiée ultrafiltrée.

On injecte une aliquote de 100 µl de chacune des solutions sur une colonne analytique CARBOPAC MAl (4 X 250 mm) (DIONEX #44066) préalablement équilibrée par une solution de soude 480 mM. On soumet la colonne à un flux d'une solution de soude 480 mM pendant 60 minutes à un débit de 0,4 ml/min pour éluer les monosaccharides neutres tels que le ribitol et le mannose. La température de la colonne est maintenue à 30°C.

Dans ces conditions, le pic de chromatographie correspondant au ribitol apparaît à 19,9 ± 5 % min tandis que le pic correspondant au mannose (étalon interne) apparaît à 25,2 ± 5 % min.

On établit alors la courbe-étalon (quantité de ribitol en fonction de la surface des pics). Par interpolation, on détermine la quantité de ribitol contenu dans la préparation de départ. Puis on déduit alors la quantité exacte de polysaccharide C5, sachant que l'unité répétitive du polysaccharide C5 est constituée de 11,1% (p/p) de ribitol.

### 1.1.2. Polysaccharide C :

On dissout la poudre de polysaccharide C purifié (Staten Serum Institute, DanemarK) dans de l'eau ultrapurifiée puis on ajuste sa concentration à 10 µg/ml sur la base du dosage du ribitol effectué selon la même méthode que celle détaillée au paragraphe 1.1.1.

### 1.2. Gammes-étalon :

De 0 à 5 µg/ml en acide téchoïques précisément dosés préparées à partir des solutions mères par dilution dans de l'eau purifiée ultrafiltrée. Les échantillons de la gamme sont ensuite séchés sous azote.

### 1.3. Préparation de l'échantillon à doser

On dialyse 3 doses de vaccins (1,5 ml) contre de l'eau distillée puis on lyophilise le dialysat. Le lyophilisat est alors repris par 1,5 ml d'eau purifiée ultrafiltrée dont on prélève une aliquote de 40 µl qui est ensuite séché sous azote.

Pour contrôler la reproductibilité de la chromatographie, on peut rajouter dans l'échantillon à analyser une quantité fixe de fructose qui sert d'étalon interne.

### 1.4. Hydrolyse par l'HF

Tous les dessicats (échantillon à doser et échantillons de la gamme étalon) sont traités par 400 µl d'acide fluorhydrique à 48 % pendant 48 heures à 5°C. On sèche sous courant d'azote et on reprend par 400 µl d'eau purifiée ultrafiltrée au moment de l'analyse.

### 1.5. Analyse par chromatographie HPAEC-PAD

On injecte 100 µl de chacun des hydrolysats sur une colonne analytique CARBOPAC PAl (4 X 250 mm) (DIONEX #35391) préalablement équilibrée par une solution de soude 75 mM. On soumet la colonne à un flux d'une solution de soude 75 mM pendant 10 min à un débit de 1 ml/min pour éluer les oligosaccharides spécifiques du polysaccharide C et du polysaccharide C5 ainsi que les monosaccharides neutres au pH de l'analyse. La température de la colonne est maintenue à 30°C. Pour parachever la chromatographie, on ajoute graduellement une solution de soude 75 mM contenant de l'acétate de sodium de façon à éluer tous les monosaccharides, oligosaccharides et polysaccharides restants.

Dans ces conditions, le pic de chromatographie correspondant au ribitol libéré lors de l'hydrolyse du polysaccharide C, du polysaccharide C5 et des polysaccharides capsulaires 6B et 10A apparaît à 2,65 ± 5% min. Les pics correspondant aux oligosaccharides spécifiques du polysaccharide C5 et du polysaccharide C ainsi que le pic de fructose (étalon interne) apparaissent respectivement à 3,30 ± 5% min, 4,30 ± 5% min et 8,5 ± 5% min (voir figure 1).

On établit les courbes-étalon (quantité de polysaccharide C ou C5 en fonction de la surface des pics). Par interpolation, on détermine les quantités de polysaccharide C et C5 présentes dans la préparation de départ.

### Exemple 2 : Dosage des quantités résiduelles en polysaccharide C et C5 dans la formulation vaccinale F3 telle que décrite dans WO 98/51339 contenant les conjugués Dt et/ou Tt des polysaccharides capsulaires des sérotypes 1, 3, 4, 5, 6B, 7F, 9V, 14, 18C, 19F et 23F du pneumocoque.

Pour préparer l'échantillon à doser on utilise le même protocole que celui décrit au paragraphe 1.3. Le volume de l'aliquote prélevée est ici de 1 ml. Le profil de chromatographie que l'on obtient est celui montré à la figure 2.

## Revendications

1. Une méthode de dosage des acides téchoïques de type polysaccharidique dans une préparation issue de culture de *Streptococcus pneumoniae* selon laquelle :
*(1)* on traite la préparation par l'acide fluorhydrique (HF) à une température inférieure ou égale à 40°C, afin de libérer les oligosaccharides spécifiques des acides téchoïques présents dans la préparation, dont 1a structure de ces oligosaccharides est dépourvue de toute liaison chimique de type phosphate ester mais est identique à l'enchaînement des sucres de l'unité répétitive des acides téchoïques ou s'en distingue par la perte du ribitol ou du glycérol lorsque la structure de l'unité répétitive renferme une molécule de β-D GalpNAc ou une molécule de β-D GlcpNAc liée à une molécule de ribitol phosphate ou de glycerol phosphate; et
*(2)* on dose les oligosaccharides spécifiques obtenus en *(1)*.

2. Une méthode selon la revendication 1, selon laquelle les acides téchoïques à doser sont le polysaccharide C et l'acide lipotéchoïque et selon laquelle les oligosaccharides spécifiques du polysaccharide C et de l'acide lipotéchoïque ont une structure chimique commune sous la forme :
β-D-Glcp-(1**→**3)-α**-**AAT*p***-**(1**→**4)-α-D-Gal*p*NAc-(1**→**3)-β-D-Gal*p*NAc

3. Une méthode de dosage selon la revendication 1 ou 2, dans laquelle la préparation est un surnageant de culture.

4. Une méthode de dosage selon la revendication 1 ou 2, dans laquelle la préparation comprend un ou plusieurs antigènes purifiés.

5. Une méthode de dosage selon l'une des revendications 1 à 4, dans laquelle on traite la préparation par l'acide fluorhydrique (HF) à une température comprise entre 0 et 20°C, bornes incluses.

6. Une méthode de dosage selon la revendication 5, dans laquelle on traite la préparation par l'acide fluorhydrique (HF) à une température comprise entre 4 et 10°C, bornes incluses.

7. Une méthode de dosage selon l'une des revendications 1 à 6, dans laquelle on traite la préparation par l'acide fluorhydrique (HF) à une concentration finale comprise entre 10 et 73 % (poids / poids), bornes incluses.

8. Une méthode de dosage selon la revendication 7, dans laquelle on traite la préparation par l'acide fluorhydrique (HF) à une concentration finale comprise entre 40 et 60 %.

9. Une méthode de dosage selon la revendication 8, dans laquelle on traite la préparation par l'acide fluorhydrique (HF) à une concentration finale comprise entre 45 et 50 %.

10. Une méthode de dosage selon l'une des revendications 1 à 9, dans laquelle on dose les oligosaccharides spécifiques par une technique permettant la séparation et/ou le dosage des monosaccharides.

11. Une méthode de dosage selon la revendication 10, dans laquelle on dose les oligosaccharides spécifiques par une technique qui est la chromatographie d'échange d'anions hautement résolutive (HPAEC), éventuellement couplée à une détection ampérométrique en champs pulsés (PAD).

12. Une méthode selon la revendication 11, dans laquelle la chromatographie HPAEC utilise un support compatible avec un pH supérieur ou égal à 12.

13. Une méthode selon la revendication 12, dans laquelle le support est constitué par du polystyrène-divinyl-benzène sulfonaté.

14. Une méthode selon la revendication 13, dans laquelle le polystyrène-divinyl-benzène sulfonaté possède un degré de réticulation de 1 à 5 %.

15. Une méthode selon la revendication 13 ou 14, dans laquelle le polystyrène-divinyl-bonzène sulfonaté se présente sous forme de microbilles ayant un diamètre de 450 à 550 nm.

16. Une méthode selon l'une des revendications 12 à 15, dans laquelle le support de chromatographie est conditionné sous forme de colonne et est complété en sa partie supérieure, par une couche d'un matériau porteur de charges positives permettant d'optimiser la résolution de la colonne.

17. Une méthode selon la revendication 16, dans laquelle le matériau permettant d'optimiser la résolution est constitué par un matériau porteur d'un sel d'amine primaire, d'un sel d'amine secondaire, d'un sel d'amine tertiaire, d'un sel d'ammonium quaternaire, ou d'un groupement ammonium.

18. Une méthode selon la revendication 17, dans laquelle le matériau est du latex possédant un degré de réticulation de 3 à 7 %.

19. Une méthode selon la revendication 11, dans laquelle la chromatographie HPAEC utilise une solution d'élution possédant un pH ≥ 12.

20. Une méthode selon la revendication 19, dans laquelle la solution d'élution est dépourvue de carbonate.

21. Une méthode selon la revendication 19 ou 20, dans laquelle la solution d'élution est une solution de soude dont la molarité est comprise entre 10 et 300 mM.

22. Une méthode selon la revendication 21, dans laquelle la solution de soude possède une molarité comprise entre 40 et 100 mM.

23. Une méthode selon l'une des revendications 1 à 22, dans laquelle la préparation comprend un ou plusieurs polysaccharides capsulaires de *Streptococcus pneumoniae* de sérotype 6B, 10A, 17F, 19A, 19F ou 20.

24. Une méthode selon la revendication 23, dans laquelle la préparation comprend les polysaccharides capsulaires des sérotypes 6B, 10A, 17F, 19A, 19F et 20 de *Streptococcus pneumoniae*.

25. Une méthode selon l'une des revendications 1 à 24, dans laquelle la préparation comprend un polysaccharide capsulaire de *Streptococcus pneumoniae* de sérotype 5 et dans laquelle l'acide téchoïque à doser est le polysaccharide C5 dont l'unité répétitive a pour formule :

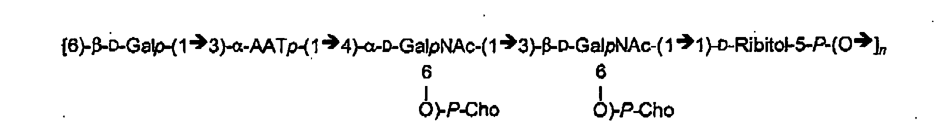

26. Polysaccharide C5 purifié possédant une unité répétitive de formule :

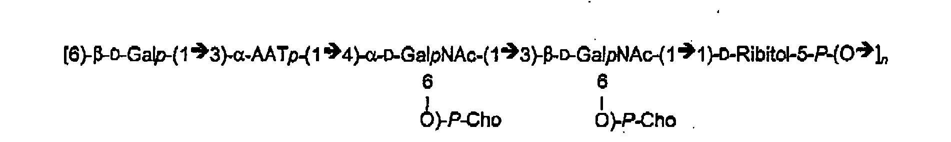

27. Utilisation du polysaccharide C5 selon la revendication 26, pour établir une courbe d'étalonnage destinée au dosage du polysaccharide C5, dans une préparation comprenant un polysaccharide capsulaire de *Streptococcus pneumoniae* de sérotype 5.

## Claims

1. Method for assaying the polysaccharide-type teichoic acids in a preparation derived from a culture of *Streptococcus pneumoniae,* according to which:
(*1*) the preparation is treated with hydrofluoric acid (HF) at a temperature of less than or equal to 40°C, in order to release the specific oligosaccharides of the teichoic acids present in the preparation, of which the structure of these oligosaccharides is devoid of any chemical bond of phosphate ester type but is identical to the chain of sugars of the teichoic acid repeating unit or differs therefrom by virtue of the loss of the ribitol or of the glycerol when the structure of the repeating unit contains a β-D GalpNAc molecule or a β-D GlcpNAc molecule linked to a ribitol phosphate or glycerol phosphate molecule; and
(2) the specific oligosaccharides obtained in (*1*) are assayed.

2. A method according to Claim 1, according to which the teichoic acids to be assayed are the C-polysaccharide and lipoteichoic acid, and according to which the C-polysaccharide-specific oligosaccharides and the lipoteichoic acid-specific oligosaccharides have a common chemical structure in the form:
β-D-Glcp-(1**→**3)-α**-**AAT*p***-**(1**→**4)-α-D-Gal*p*NAc-(1**→**3)-β-D-Gal*p*Nc

3. Assaying method according to Claim 1 or 2, in which the preparation is a culture supernatant.

4. Assaying method according to Claim 1 or 2, in which the preparation comprises one or more purified antigens.

5. Assaying method according to one of Claims 1 to 4, in which the preparation is treated with hydrofluoric acid (HF) at a temperature of between 0 and 20°C, limits inclusive.

6. Assaying method according to Claim 5, in which the preparation is treated with hydrofluoric acid (HF) at a temperature of between 4 and 10°C, limits inclusive.

7. Assaying method according to one of Claims 1 to 6, in which the preparation is treated with hydrofluoric acid (HF) at a final concentration of between 10 and 73% (weight/weight), limits inclusive.

8. Assaying method according to Claim 7, in which the preparation is treated with hydrofluoric acid (HF) at a final concentration of between 40 and 60%.

9. Assaying method according to Claim 8, in which the preparation is treated with hydrofluoric acid (HF) at a final concentration of between 45 and 50%.

10. Assaying method according to one of Claims 1 to 9, in which the specific oligosaccharides are assayed using a technique which makes it possible to separate and/or to assay the monosaccharides.

11. Assaying method according to Claim 10, in which the specific oligosaccharides are assayed using a technique which is high performance anion exchange chromatography (HPAEC), optionally coupled with pulsed amperometric detection (PAD).

12. Method according to Claim 11, in which the HPAEC uses a support compatible with a pH greater than or equal to 12.

13. Method according to Claim 12, in which the support consists of sulfonated polystyrene-divinylbenzene.

14. Method according to Claim 13, in which the sulfonated polystyrene-divinylbenzene has a degree of crosslinking of 1 to 5%.

15. Method according to Claim 13 or 14, in which the sulfonated polystyrene-divinylbenzene is in the form of microbeads having a diameter of 450 to 550 nm.

16. Method according to one of Claims 12 to 15, in which the chromatography support is packaged in the form of a column and is completed, in its upper part, by a layer of a material bearing positive charges for optimizing the column resolution.

17. Method according to Claim 16, in which the material for optimizing the resolution consists of material bearing a primary amine salt, a secondary amine salt, a tertiary amine salt, a quaternary ammonium salt, or an ammonium group.

18. Method according to Claim 17, in which the material is latex having a degree of crosslinking of 3 to 7%.

19. Method according to Claim 11, in which the HPAEC uses an eluting solution having a pH ≥ 12.

20. Method according to Claim 19, in which the eluting solution is free of carbonate.

21. Method according to Claim 19 or 20, in which the eluting solution is a sodium hydroxide solution, the molarity of which is between 10 and 300 mM.

22. Method according to Claim 21, in which the sodium hydroxide solution has a molarity of between 40 and 100 mM.

23. Method according to one of Claims 1 to 22, in which the preparation comprises one or more capsular polysaccharides of *Streptococcus pneumoniae* serotype 6B, 10A, 17F, 19A, 19F or 20.

24. Method according to Claim 23, in which the preparation comprises the capsular polysaccharides of *Streptococcus pneumoniae* serotypes 6B, 10A, 17F, 19A, 19F and 20.

25. Method according to one of Claims 1 to 24, in which the preparation comprises a capsular polysaccharide of *Streptococcus pneumoniae* serotype 5 and in which the teichoic acid to be assayed is the C5-polysaccharide of which the repeating unit has the formula:

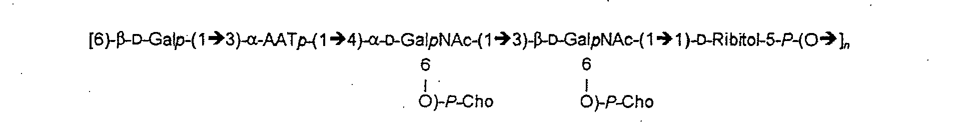

26. Purified C5-polysaccharide having a repeating unit of formula:

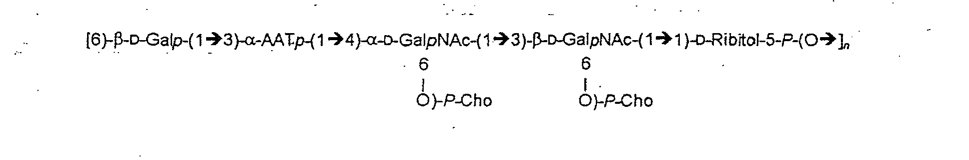

27. Use of the C5-polysaccharide according to Claim 26, for establishing a standard curve intended for assaying the C5-polysaccharide, in a preparation comprising a capsular polysaccharide of *Streptococcus pneumoniae* serotype 5.

## Patentansprüche

1. Verfahren zur Bestimmung von Teichonsäuren vom Typ Polysaccharid in einer Präparation, die von der Kultur von *Streptococcus pneumoniae* stammt, gemäß der man:
(1) die Präparation mit Fluorwasserstoffsäure (HF) bei einer Temperatur von unter oder gleich 40 °C behandelt, um die in der Präparation vorliegenden spezifischen Oligosaccharide der Teichonsäuren freizusetzen, wobei die Struktur von diesen Oligosacchariden von jeder chemischen Bindung vom Typ Phosphatester frei ist aber identisch mit der Verkettung von Zuckern von wiederholten Einheiten von Teichonsäuren ist, oder sich darin durch den Verlust von Ribitol oder von Glycerol unterscheidet, wenn die Struktur der wiederholten Einheiten ein Molekül von β-D GalpNAc oder ein Molekül von β-D GlcpNAc, gebunden an ein Molekül von Ribitolphosphat oder von Glycerolphosphat, umfasst, und
(2) die in (1) erhaltenen spezifischen Oligosaccharide bestimmt.

2. Verfahren nach Anspruch 1, gemäß dem die zu bestimmenden Teichonsäuren das Polysaccharid C und die Lipoteichonsäure sind und gemäß dem die spezifischen Oligosaccharide des Polysaccharides C und der Lipoteichonsäure eine gemeinsame chemische Struktur unter der Form:
β-D-Glcp-(**1→3**)-α**-**AAT*p***-**(**1→4**)-α-D-Gal*p*NAc-(**1→3**)-β-D-Gal*p*Nc
besitzen.

3. Verfahren zur Bestimmung nach Anspruch 1 oder 2, bei dem die Präparation ein aufschwimmender Anteil der Kultur ist.

4. Verfahren zur Bestimmung nach Anspruch 1 oder 2, bei dem die Präparation ein oder mehrere gereinigte Antigene umfasst.

5. Verfahren zur Bestimmung nach einem der Ansprüche 1 bis 4, bei dem man die Präparation mit Fluorwasserstoffsäure (HF) bei einer Temperatur zwischen einschließlich 0 °C und 20 °C behandelt, Grenzwerte eingeschlossen.

6. Verfahren zur Bestimmung nach Anspruch 5, bei dem man die Präparation mit Fluorwasserstoffsäure (HF) bei einer Temperatur zwischen einschließlich 4 °C und 10 °C behandelt, Grenzwerte eingeschlossen.

7. Verfahren zur Bestimmung nach einem der Ansprüche 1 bis 6, bei dem man die Präparation mit Fluorwasserstoffsäure (HF) mit einer Endkonzentration zwischen einschließlich 10 % und 73 % (Gewicht/Gewicht) behandelt, Grenzwerte eingeschlossen.

8. Verfahren zur Bestimmung nach Anspruch 7, bei dem man die Präparation mit Fluorwasserstoffsäure (HF) mit einer Endkonzentration zwischen einschließlich 40 % und 60 % behandelt.

9. Verfahren zur Bestimmung nach Anspruch 8, bei dem man die Präparation mit Fluorwasserstoffsäure (HF) mit einer Endkonzentration zwischen einschließlich 45 % und 50 % behandelt.

10. Verfahren zur Bestimmung nach einem der Ansprüche 1 bis 9, bei dem man die spezifischen Oligosaccharide durch eine Technik bestimmt, welche die Abtrennung und/oder Bestimmung von Monosacchariden ermöglicht.

11. Verfahren zur Bestimmung nach Anspruch 10, bei dem man die spezifischen Oligosaccharide durch eine Technik bestimmt, welche die hochauflösende Anionen-Austausch-Chromatographie (HPAEC) ist, gegebenenfalls gekoppelt mit einer amperometrischen Detektion im gepulsten Feld (PAD).

12. Verfahren nach Anspruch 11, bei dem die Chromatographie HPAEC einen Träger verwendet, der mit einem pH-Wert von über oder gleich 12 kompatibel ist.

13. Verfahren nach Anspruch 12, bei dem der Träger aus Polystyrol-divinyl-benzol-sulfonat besteht.

14. Verfahren nach Anspruch 13, bei dem das Polystyrol-divinyl-benzol-sulfonat einen Vernetzungsgrad von 1 bis 5 % besitzt.

15. Verfahren nach Anspruch 13 oder 14, bei dem das Polystyrol-divinyl-benzol-sulfonat in Form von Mikrokügelchen mit einem Durchmesser von 450 bis 550 nm vorliegt.

16. Verfahren nach einem der Ansprüche 12 bis 15, bei dem der Chromatographie-Träger in Kolonnenform konditioniert und an seinem oberen Teil durch eine Schicht aus einem positive Ladungen tragenden Material vervollständigt wird, das eine Optimierung der Auflösung der Kolonne ermöglicht.

17. Verfahren nach Anspruch 16, bei dem das Material, das die Optimierung der Auflösung ermöglicht, aus einem Material besteht, das Träger eines Salzes von einem primären Amin, eines Salzes von einem sekundären Amin, eines Salzes von einem tertiären Amin, eines quaternären Ammoniumsalzes oder einer Ammoniumgruppe ist.

18. Verfahren nach Anspruch 17, bei dem das Material ein Latex ist, der einen Vernetzungsgrad von 3 bis 7 % besitzt.

19. Verfahren nach Anspruch 11, bei dem die Chromatographie HPAEC eine Elutionslösung verwendet, die einen pH-Wert ≥ 12 besitzt.

20. Verfahren nach Anspruch 19, bei dem die Elutionslösung frei von Carbonat ist.

21. Verfahren nach Anspruch 19 oder 20, bei dem die Elutionslösung eine Lösung von Natriumhydroxid ist, deren Molarität zwischen einschließlich 10 und 300 mM beträgt.

22. Verfahren nach Anspruch 21, bei dem die Lösung von Natriumhydroxid eine Molarität zwischen einschließlich 40 und 100 mM besitzt.

23. Verfahren nach einem der Ansprüche 1 bis 22, bei dem die Präparation ein oder mehrere verkapselte Polysaccharide von *Streptococcus pneumoniae* vom Serotyp 6B, 10A, 17F, 19A, 19F oder 20 umfasst.

24. Verfahren nach Anspruch 23, bei dem die Präparation die verkapselten Polysaccharide der Serotypen 6B, 10A, 17F, 19A, 19F und 20 von *Streptococcus pneumoniae* umfasst.

25. Verfahren nach einem der Ansprüche 1 bis 24, bei dem die Präparation ein verkapseltes Polysaccharid von *Streptococcus pneumoniae* vom Serotyp 5 umfasst, und bei dem die zu bestimmende Teichonsäure das Polysaccharid C5 ist, dessen wiederholte Einheit die folgende Formel besitzt:

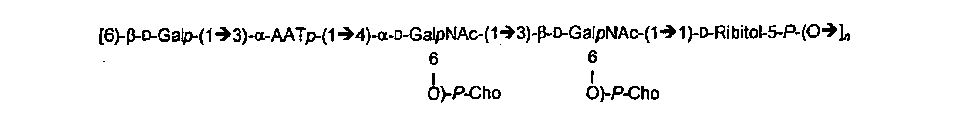

26. Gereinigtes Polysaccharid C5, das eine wiederholte Einheit der folgende Formel besitzt:

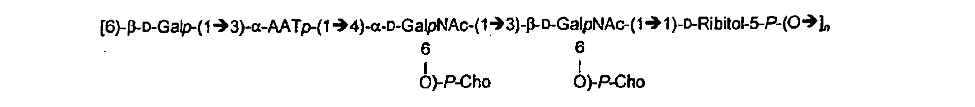

27. Verwendung des Polysaccharides C5 nach Anspruch 26 für die Einrichtung einer Eichkurve, vorgesehen zur Bestimmung des Polysaccharides C5 in einer Präparation, die ein verkapseltes Polysaccharid von *Streptococcus pneumoniae* vom Serotyp 5 umfasst.
